# EUROPEAN PATENT APPLICATION

(11) **EP 4 843 564 A1**
(43) Date of publication of application: **25.11.2026**
(21) Application number: 26180282.1
(22) Date of filing: 21.05.2026
(51) Int. Cl.: A61K 9/08, A61K 31/4985, A61K 47/02, A61K 47/12, A61K 47/26

(54) **STABLE ORAL SOLUTION OF SITAGLIPTIN AND METHODS OF PREPARATION**

(30) Priority: 22.05.2025 US 202519216304
(71) Applicant: Saptalis Pharmaceuticals, LLC, Hauppauge New York 11788 (US)
(72) Inventor: Kuznetsova, Larisa, Port Jefferson, 11777 (US); Dondeti, Polireddy, Centerport, 11721 (US); Orman, Eleonora, Oakland Gardens, 11364 (US); Sinha, Ayush, Stony Brook, 11790 (US); Naguboyina, Tejaswini, Smithtown, 11787 (US)
(74) Representative: Turner, Craig Robert

(57) **Abstract**

The present invention relates to a stable pharmaceutical oral solution composition comprising Sitagliptin or a pharmaceutically acceptable salt thereof, at a concentration of 25 mg per 5 mL. The formulation is designed to exhibit improved stability for at least 24 months at room temperature (25°C ± 2°C), maintaining an active pharmaceutical ingredient (API) potency of at least 99% w/v±2% w/v, with total degradation product levels not exceeding 0.6% w/v. The composition further comprises a preservative, a sweetener, a flavoring agent, and optionally one or more chelating agents, antioxidants, and wetting agents, along with a pH-adjusting agent and purified water, while being free from sugar alcohols. The formulation maintains a pH range of 2.0 to 5.0, preferably from about 2.5 to 4.5, and most preferably from about 3.3 to 3.7 to ensure optimal stability and is stored in light-protective containers to minimize API degradation. The invention also discloses a method for preparing the stable solution, which includes dissolving excipients in purified water under continuous stirring, incorporating Sitagliptin phosphate under controlled temperature (20-35°C), homogenizing the mixture, adjusting pH as necessary, and filtering the solution through a 1-10 µm pore filter before filling into light-protective containers, such as amber glass bottles or high-density polyethylene (HDPE) bottles. The formulation is tested for chemical stability using High-Performance Liquid Chromatography (HPLC) with a detection limit of 0.025% w/v and a quantitation limit of 0.05% w/v. The invention provides a stable, effective, and patient-friendly oral solution of Sitagliptin, offering advantages over conventional formulations in terms of long-term storage stability and ease of administration.

## Description

### FIELD OF THE INVENTION

The present invention relates to an oral pharmaceutical composition of Sitagliptin or its pharmaceutically acceptable salts, particularly an oral solution formulation comprising Sitagliptin salt and a method for its preparation. The invention pertains to an improved liquid formulation with stability at room temperature for 24 months and palatability, suitable for the treatment of type 2 diabetes mellitus.

### BACKGROUND OF THE INVENTION

Diabetes mellitus is a chronic metabolic disorder characterized by impaired glucose regulation, often necessitating pharmacological intervention to maintain glycaemic control. One of the therapeutic approaches for diabetes management involves the inhibition of dipeptidyl peptidase-IV (DPP-IV), an enzyme that catalyzes the inactivation of glucagon-like peptide-1 (GLP-1). By inhibiting DPP-IV, gliptins (DPP-IV inhibitors) increase endogenous GLP-1 levels, thereby enhancing insulin secretion and improving the glycaemic profile of patients.

Currently, commercially available gliptins, including sitagliptin, vildagliptin, saxagliptin, teneligliptin, alogliptin, and linagliptin, are primarily formulated as conventional solid oral dosage forms such as tablets. While effective, tablet formulations may present challenges in terms of patient compliance, particularly among paediatric and geriatric populations, as well as those with dysphagia or difficulty swallowing solid dosage forms. Oral liquid formulations offer an alternative that enhances patient compliance through ease of administration, more reproducible bioavailability, and the potential for flexible dosing regimens based on body weight or surface area.

However, the development of oral liquid gliptin formulations presents several challenges, particularly concerning stability and taste-masking. Gliptins are inherently bitter, necessitating effective taste-masking strategies to ensure patient acceptability and adherence. Additionally, maintaining the chemical and microbial stability of gliptins in aqueous solutions is crucial to achieving an extended shelf life and practical storage conditions.

Several prior art formulations have attempted to address the delivery of DPP-IV inhibitors, but they do not fully resolve the challenges associated with oral liquid gliptin compositions, particularly in terms of stability and taste-masking. For instance, EP1532149 discloses an aqueous composition comprising a substituted xanthine as a DPP-IV inhibitor and hydroxypropyl cellulose as a thickener, formulated for intravenous suspension or solid dosage forms such as coated tablets, capsules, powders, or suppositories. However, it does not address oral liquid solutions containing a gliptin or the associated issues of bitter taste. Similarly, WO2007/078726 discloses a pharmaceutical composition comprising a DPP-IV inhibitor in combination with 25-94 %w/v metformin hydrochloride, but it does not pertain to liquid formulations.

Among liquid formulations, EP1354882 describes a DPP-IV inhibitor in an oral syrup containing water, sugar, sorbitol, fructose, glucose, oil, an antiseptic, and a flavoring agent. EP1828192 discloses an oral formulation containing a non-gliptin DPP-IV inhibitor with citric acid, sodium hydroxide, and a flavoring agent, but it does not address gliptins specifically. WO2015/071859 describes an oral formulation of a DPP-IV inhibitor that disintegrates within three minutes of oral administration, differing from true liquid formulations. More recent patents, such as US20220387427A1 and US20240285619A1, disclose aqueous oral gliptin compositions containing a gliptin or a pharmaceutically acceptable salt or ester thereof, along with a thickening agent, an artificial non-sugar alcohol sweetening agent, an antioxidant, a buffering agent, and a preservative. These formulations have a sugar alcohol content of less than 20 % w/v and a pH range of 3 to 8, yet they still face stability concerns.

Recent approvals, such as BRYNOVIN^{™} (sitagliptin oral solution), demonstrate regulatory acceptance of liquid gliptin formulations. However, these formulations also highlight key challenges, particularly concerning stability and storage requirements, which must be addressed for broader commercial viability. Many existing liquid gliptin formulations require refrigeration (e.g., storage at 2°C to 8°C), which limits their practicality in real-world settings. Furthermore, maintaining the integrity of gliptins in solution over an extended shelf life remains a formulation challenge, particularly with respect to pH control, microbial preservation, and excipient interactions.

Given these limitations, there remains a need for an improved oral liquid gliptin formulation that ensures enhanced stability, effective taste-masking, and extended shelf life while maintaining therapeutic efficacy. The present invention meets these needs by providing a novel composition that overcomes the drawbacks of prior formulations, offering a patient-friendly and commercially viable alternative for diabetes management.

### SUMMARY OF THE INVENTION

The present invention relates to a stable pharmaceutical oral solution composition comprising Sitagliptin or its pharmaceutically acceptable salt at a concentration of 25 mg per 5 mL. The composition exhibits improved stability for at least 24 months at room temperature (25°C ± 2°C) while maintaining API potency of at least 99%±2% w/v and degradation product levels with the total impurities not exceeding 0.6% w/v.

To achieve optimal stability, the formulation comprises a preservative, a sweetener, a flavoring agent, a pH-adjusting agent, and purified water, optionally chelating agents, antioxidants and wetting agents, while being free from sugar alcohols. Preferably, sodium benzoate is used as a preservative, sucralose as a sweetener, and strawberry flavor as a flavoring agent, optionally disodium edetate salt as a chelating agent, butylated hydroxyl anisole (BHA) or metabisulfite as antioxidant and polysorbate 80 as a wetting agent. The pH is maintained between 2.0 to 5.0, and more preferably between 2.5-4.5, and most preferably from about 3.3 to 3.7 using suitable buffering agent.

The solution is stored in light-protective containers, such as HDPE bottles, to prevent API degradation. The formulation remains free from visible precipitation or turbidity throughout its shelf life.

The invention further provides a method for preparing the stable oral solution, which involves dissolving a preservative, sweetener, and flavoring agent in purified water under continuous stirring, followed by the optional addition of a wetting agent. A pH agent or a portion thereof is then incorporated, after which Sitagliptin phosphate is admixed under controlled temperatures (20-35°C). The mixture may optionally be homogenized to ensure uniform dissolution and homogeneity. If required, the pH is adjusted using a pH-adjusting agent, and the final volume is brought to the desired level by adding the remaining water. The solution is then optionally filtered through a 1 to 10 µm pore filter before being filled into an appropriate container.

Preferably, the preparation process is conducted at 15°C to 25°C, followed by 0.2-micron filtration to remove particulate matter. The solution is then filled into light-protective containers under nitrogen flushing to further enhance stability. Chemical stability is assessed using High-Performance Liquid Chromatography (HPLC) with a detection limit of 0.025% w/v. and quantitation limit of 0.05% w/v.

This invention provides a sugar alcohol-free, stable, and pharmaceutically elegant Sitagliptin oral solution with extended shelf life, making it an improved alternative for diabetes management.

### BRIEF DESCRIPTIONS OF THE TABLES

**Table 1: Stability Data for Sitagliptin after 1 week at 60°C**
   Short-term accelerated stability data evaluating Sitagliptin assay and impurity formation under high-temperature conditions.
**Table 2: Stability Data for Sitagliptin after 1 month at 40°C**
   Stability data after one month at 40°C.
**Table 3: Stability Data for Sitagliptin after 2 months at 40°C**
   Stability data after two months at 40°C.
**Table 4: Stability Data for Sitagliptin after 1 month at RT**
   Stability data at room temperature for one month, assessing the effect of different stabilizing excipients on Sitagliptin assay and impurity formation.
**Table 5: Stability Data for Sitagliptin after 2 months at RT**
   Stability data at room temperature for two months, assessing the effect of different stabilizing excipients on Sitagliptin assay and impurity formation.
**Table 6: Stability Data for Sitagliptin after 18 months at RT and 25°C/60% Relative Humidity (RH)**
   Long-term stability data collected at controlled conditions (25°C/60% RH), validating the formulation's stability profile over an extended period.
**Table 7: Sensory Evaluation of Flavoring Agents**
   Presents the results of a sensory evaluation conducted on various flavoring agents.

### BRIEF DESCRIPTION OF THE DRAWINGS

Figure 1 is a graph of Predicted Formation of Sitagliptin Acid Impurity in batch no 75.2 at RT up to 24 months
Figure 2 is a graph of Predicted Formation of Sitagliptin Acid Impurity in batch no 02.3 at RT up to 24 months

### DETAILED DESCRIPTION OF THE INVENTION

The present invention effectively addresses the challenges associated with oral liquid formulations of sitagliptin by incorporating a scientifically optimized composition that ensures both stability and patient acceptability. The composition maintains sitagliptin or its pharmaceutically acceptable salt at a concentration of 25 mg per 5 mL while achieving long-term stability of at least 24 months at room temperature (25°C ± 2°C) without compromising API potency or exceeding degradation product levels of 0.6% w/v. This enhanced stability is achieved through precise pH modulation within the range of 2.5 to 4.5 using hydrochloric acid, which minimizes hydrolytic and oxidative degradation.

Additionally, the exclusion of sugar alcohols eliminates potential gastrointestinal discomfort, while sucralose effectively masks bitterness without impacting stability. The inclusion of a preservative ensures microbial integrity, further supporting extended shelf life. Moreover, the formulation remains free from visible precipitation or turbidity over time, owing to the controlled selection of excipients and a filtration step. To mitigate light-induced degradation, the composition is stored in light-protective HDPE containers under nitrogen flushing. These advancements collectively establish a novel, commercially viable, and patient-friendly alternative to conventional oral sitagliptin formulations, addressing key barriers to oral liquid gliptin development.

The term "Pharmaceutical Oral Solution Composition" as herein used refers to a liquid dosage form containing an active pharmaceutical ingredient (API) dissolved in a suitable solvent for oral administration.

Sitagliptin, a dipeptidyl peptidase-4 (DPP-4) inhibitor used for treating type 2 diabetes mellitus, can be present as a pharmaceutically acceptable salt to enhance solubility and stability. As used herein, the term "pharmaceutically acceptable salts of sitagliptin" refers to salts formed from sitagliptin with inorganic or organic acids that are suitable for pharmaceutical use and do not adversely affect the biological activity, safety, or stability of the compound. Such salts include, but are not limited to, hydrochloride, sulfate, phosphate, nitrate, maleate, fumarate, citrate, tartrate, mesylate, besylate, malate, succinate, acetate, oxalate, lipoate, orotate, caffeate, coumarate, and ferulate salts. These salts may exist in anhydrous or hydrated forms and may further exist as crystalline or amorphous solids. Preferred pharmaceutically acceptable salts include sitagliptin phosphate monohydrate, sitagliptin hydrochloride, sitagliptin fumarate, sitagliptin tartrate and sitagliptin maleate due to their favorable solubility and stability profiles. The salts may be prepared by conventional techniques, such as reacting sitagliptin free base with the appropriate acid in a suitable solvent, followed by isolation using standard purification procedures known in the art.

All concentrations or amounts of sitagliptin mentioned herein, unless otherwise specified, refer to the sitagliptin free base equivalent.

The oral pharmaceutical composition of the present invention comprises Sitagliptin in an amount ranging from about 0.25% to about 3.5% w/v, preferably 25mg/5mL.

A preservative is added to prevent microbial growth and ensure stability. The preservative is selected from methyl paraben, ethyl paraben, propyl paraben, butyl paraben, benzoic acid, sodium benzoate, benzyl alcohol, sorbic acid, potassium sorbate, and combinations thereof.

The preferred preservative comprises sodium benzoate or benzoic acid in an amount ranging from about 0.05% to about 0.2% w/v, more preferably from about 0.075% to about 0.15% w/v, and most preferably around 0.1% w/v.

A non-sugar alcohol sweetener is used to enhance taste while avoiding the thickening effect and gastrointestinal side effects of sugar alcohols. The preferred sweetener is selected from artificial non-sugar alcohol sweetening agents selected from sucralose, sodium saccharin, acesulfame-K, aspartame, alitame, cyclamate, stevioside, glycyrrhizin, neo hesperidin, dihydrochalcone, thaumatin, or combinations thereof. Preferably sucralose in a concentration of 0.1 - 2.0 % w/v, more preferably from about 0.15% to about 1.0% w/v, and most preferably from about 0.5% to about 0.7% w/v.

A flavoring agent is added to mask the taste of sitagliptin and improve palatability. The preferred flavoring agents include natural or synthetic flavours such as banana, lemon, orange, grape, lime, grapefruit, vanilla, bubble gum, peppermint, fantasy fruit masking flavour, and fruit essences including apple, banana, pear, peach, strawberry, raspberry, cherry, plum, pineapple, and apricot. Other examples include synthetic flavour oils, flavoring aromatics, or natural oils like cinnamon oil, oil of wintergreen, peppermint oil, clove oil, citrus oil, bay oil, anise oil, eucalyptus, thyme oil, cedar leaf oil, oil of nutmeg, oil of sage, oil of bitter almonds, cassia oil, maltol, ethyl vanillin, menthol, citric acid, fumaric acid, ethyl maltol, tartaric acid, or combinations thereof, preferably strawberry flavor in a concentration about 0.1% to about 2.0% w/v, preferably from about 0.3% to about 0.6% w/v, and most preferably from about 0.35% to about 0.45% w/v.

A wetting agent is a surfactant that reduces surface tension between a liquid and a solid, improving the dispersion of hydrophobic compounds in aqueous formulations. It ensures uniform distribution and enhances the solubility of active pharmaceutical ingredients (APIs). Examples of wetting agents include sodium lauryl sulfate, sorbitan esters of fatty acids such as sorbitan monooleate and sorbitan monostearate, ethylene oxide-propylene oxide block copolymers, lecithins, polyoxyethylated hydrogenated castor oil, and polysorbate 80, with polysorbate 80 being preferred. The concentration typically ranges from 0.01 - 0.5% w/v for polysorbate 80.

A chelating agent binds metal ions, preventing oxidative degradation and stabilizing pharmaceutical compositions, thereby enhancing their shelf life. Common chelating agents include disodium edetate (EDTA), citric acid, malic acid, and tartaric acid, with EDTA being preferred. The concentration typically ranges from 0.01 - 0.1% w/v, preferably 0.05% w/v for EDTA.

An antioxidant protects the API and excipients from oxidative degradation caused by exposure to oxygen or light, improving formulation stability and efficacy. Examples of antioxidants include butylated hydroxytoluene (BHT), butylated hydroxyanisole (BHA), sodium metabisulfite, tocopherol, ascorbic acid and its derivatives such as ascorbyl palmitate, propyl gallate, and sodium thiosulfate, with BHA and sodium metabisulfite being preferred. The concentration typically ranges from 0.01 - 0.10% w/v, preferably 0.02 - 0.05% w/v for BHA or sodium metabisulfite.

A viscosity-enhancing agent such as xanthan gum may be included in an amount ranging from 0.01% to 0.2% w/v, preferably from 0.05% to 0.1% w/v, and most preferably around 0.01% to 0.02% w/v.

A pH-adjusting agent is a compound used to modify and stabilize the pH of the solution to optimize API stability. The pH-adjusting agent is preferably selected from hydrochloric acid, nitric acid, phosphoric acid, sulphuric acid, acetic acid, citric acid, malic acid, lactic acid, propionic acid, tartaric acid, ammonia solutions, monoethanolamine, diethanol-amine, triethanolamine, meglumine, sodium acetate, sodium citrate, potassium bicarbonate, potassium citrate, potassium hydroxide, sodium bicarbonate, sodium borate, sodium hydroxide or combinations thereof, preferably Hydrochloric acid.

The pH of the composition is preferably between 2.0-5.0, and more preferably between 2.5-4.5, and most preferably from about 3.3 to 3.7.

A coloring agent is an additive used in pharmaceutical formulations to provide a distinct color, improve aesthetic appeal, and facilitate product identification. Pharmaceutically acceptable coloring agents include natural colorants, juice concentrates, and pigments such as titanium dioxide, iron oxide, and zinc oxide.

The term antidiabetic drug refers to a pharmaceutical agent used to manage and treat diabetes by regulating blood glucose levels. These drugs function through various mechanisms, such as enhancing insulin secretion, improving insulin sensitivity, inhibiting glucose absorption, or reducing hepatic glucose production. Antidiabetic drugs can be classified into different categories, including insulin secretagogues (e.g., sulfonylureas, meglitinides), insulin sensitizers (e.g., biguanides, thiazolidinediones), α-glucosidase inhibitors, GLP-1 receptor agonists, DPP-IV inhibitors, and SGLT2 inhibitors. Examples include metformin, glibenclamide, gliclazide, glimepiride, pioglitazone, rosiglitazone, liraglutide, sitagliptin, vildagliptin, canagliflozin, and dapagliflozin, among others.

Purified water refers to water that meets pharmaceutical purity standards and is used as the primary solvent in the formulation, such as USP-grade purified water for oral solutions.

A light-protective container is designed to minimize degradation due to light exposure. Examples include HDPE (High-Density Polyethylene) bottles and amber glass bottles.

Filtration through a 1-10 micron filter is performed to remove particulate matter and ensure microbial purity, preferably through membrane filtration using a 0.2-micron filter.

High-Performance Liquid Chromatography (HPLC) testing is used to analyse the chemical stability and degradation profile of the formulation.

The gliptin is preferably dissolved in the composition as described herein, more preferably completely dissolved. The composition is preferably a solution.

The term "Room Temperature" (RT) refers to a controlled environment between 20°C and 25°C (68°F to 77°F), with allowable excursions between 15°C and 30°C (59°F to 86°F), as per ICH (International Council for Harmonisation) guidelines.

According to ICH Q1A (R2) guidelines, long-term stability testing is conducted under specific conditions based on the climatic zone:
- Zone I & II (Temperate/ Mediterranean Climate): 25°C ± 2°C / 60% RH ± 5% RH for 12-60 months
- Zone III (Hot & Dry Climate): 30°C ± 2°C / 35% RH ± 5% RH for 12-60 months
- Zone IVa (Hot & Humid Climate): 30°C ± 2°C / 65% RH ± 5% RH for 12-60 months
- Zone IVb (Very Hot & Humid Climate, e.g., ASEAN countries): 30°C ± 2°C / 75% RH ± 5% RH for 12-60 months

For most pharmaceutical products intended for worldwide distribution, the standard long-term stability condition is 25°C ± 2°C / 60% RH ± 5% RH for 12 to 60 months. However, for products targeted at tropical regions, 30°C ± 2°C / 75% RH ± 5% RH is often required.

For practical commercial use, pharmaceutical formulations should have a shelf life of at least 18 months, and preferably at least 24 months. Preferably, the pharmaceutical compositions remain stable at room temperature and do not require refrigeration. The sitagliptin compositions of the present invention maintain at least 99%±2% w/v sitagliptin content after 18 months of storage at 25°C and 60% relative humidity, and preferably at least 99%±2% w/v sitagliptin after 24 months under the same conditions.

Sitagliptin compositions of the invention provide less than 0.6% w/v total impurities after 24 months at 25°C and 60% relative humidity.

The formulation is packaged in light-protective HDPE containers flushed with nitrogen to prevent light-induced degradation. The selected excipients contribute to maintaining long-term stability, ensuring a commercially viable and patient-friendly liquid formulation of sitagliptin.

The Sitagliptin oral solution (25 mg/5 mL) was developed through extensive research aimed at optimizing stability, reducing impurity formation, and enhancing palatability.

The formulation components and their concentrations were carefully selected based on solubility, preservative efficacy, and taste-masking properties. Short-term and long-term stability studies confirmed that maintaining pH between 3.0 and 3.9 resulted in minimal impurity formation. Throughout development, the following key impurities were closely monitored:
- **Sitagliptin Acid:** (*R*)-3-Amino-4-(2,4,5-trifluorophenyl)butanoic acid,
- **Defluoro Sitagliptin (referred as Impurity-B):** (R)-3-Amino-4-(2,5-difluorophenyl)-1-(3-(trifluoromethyl)-5,6-dihydro-[1,2,4]triazolo[4,3-a]pyrazin-7(8H)-yl)butan-1-one,
- **Sitagliptin Phenylcrotonyl Analog:** (*E*)-1-{3-(Trifluoromethyl)-5,6-dihydro-[1,2,4]triazolo[4,3-*a*]pyrazin-7(8*H*)-yl}-4-(2,4,5-trifluorophenyl)but-2-en-1-one,
- **Sitagliptin Styrylacetyl Analog:** (*E*)-1-{3-(Trifluoromethyl)-5,6-dihydro[1,2,4]triazolo[4,3-*a*]pyrazin-7(8*H*)-yl}-4-(2,4,5-trifluorophenyl)but-3-en-1-one,
- **Sitagliptin (R)-Triazecine Analog** (referred as Triazine),Analog

**Table 1: Stability Data for Sitagliptin after 1 week at 60°C**

| **Batch No.** | **Additional Stabilizing Excipient** | **pH** | **Sitagliptin Assay** | **Total Impurities** | **Sitagliptin Acid** | **Impurity-B** | **Sitagliptin Phenylcrotonyl Analog** | **Sitagliptin Styrylacetyl Analog** |
|---|---|---|---|---|---|---|---|---|
| **Specification (%w/v)** | | | **90.0 - 110.0** | **NMT 0.6** | **NMT 0.2** | **NMT 0.2** | **NMT 0.2** | **NMT 0.2** |
| **75.1** | HCl | 3.3 | 102.5 | 0.225 | 0.127 | ND | ND | ND |
| **75.2** | HCl | 3.5 | 102.7 | 0.228 | 0.137 | ND | ND | ND |
| **75.3** | HCl | 3.7 | 102.1 | 0.311 | 0.171 | ND | 0.048 | ND |
| **75.4** | HCl | 3.9 | 101.7 | 0.423 | 0.228 | ND | 0.08 | ND |
| **75.5** | HCl/Xanthan Gum | 3.5 | 100.9 | 0.241 | 0.143 | ND | ND | ND |
| **83** | KHCO₃/H₃PO₄ | 3.5 | 101.0 | 0.222 | 0.166 | ND | ND | ND |
| **94** | KHCO₃/H₃PO₄ | 4.5 | 99.2 | 0.834 | 0.590 | ND | 0.244 | ND |
| **138. 1** | KHCO₃/HCl | 3.5 | 99.8 | 0.261 | 0.147 | ND | 0.026 | ND |
| **138. 2** | H₃PO₄ | 3.5 | 99.6 | 0.321 | 0.170 | ND | 0.035 | ND |
| **138. 3** | Citric Acid | 3.5 | 99.4 | 0.640 | 0.150 | 0.190 | ND | ND |
| **138. 4** | Malic Acid | 3.5 | 98.8 | 0.351 | 0.151 | 0.045 | 0.029 | ND |
| **138. 5** | HCl | 4.5 | 98.8 | 1.086 | 0.728 | ND | 0.359 | ND |
| **138. 6** | NaEDTA/H Cl | 3.5 | 99.7 | 0.233 | 0.147 | ND | 0.040 | ND |
| **138. 7** | Glycerin | 3.5 | 99.0 | 0.299 | 0.164 | ND | 0.055 | ND |
| **138. 8** | Sorbitol | 3.5 | 99.4 | 0.211 | 0.167 | ND | 0.045 | ND |
| **138. 9** | Sorbitol | 4.5 | 98.1 | 1.188 | 0.764 | ND | 0.379 | ND |
| **114** | HCl | 3.0 | 95.8 | 0.119 | 0.104 | ND | ND | ND |
| **117** | HCl | 2.5 | 99.8 | 0.280 | 0.188 | ND | ND | ND |
| **122** | HCl | 2.0 | 99.6 | 0.642 | 0.532 | ND | ND | ND |
| **125** | HCl | 5.0 | 96.7 | 2.113 | 1.430 | ND | 0.682 | ND |

The stability data for Sitagliptin after one week at 60°C indicates that formulations with a pH range of 3.3-3.5 exhibited the best stability, with minimal impurity formation. HCl-based formulations maintained Sitagliptin assay values within the acceptable range (90.0%-110.0% w/v) and showed total impurities well below 0.6% w/v, making them the most stable. NaEDTA-containing formulations, particularly Batch 138.6 (pH 3.5), exhibited excellent stability with negligible impurity formation. However, as the pH increased to 4.5 and above, impurity levels rose significantly, indicating that higher pH conditions accelerate degradation. Batch 94 (KHCO₃/H₃PO₄, pH 4.5) had elevated impurity levels (0.834% w/v, with Sitagliptin Acid at 0.590% w/v), while Batch 138.9 (10% Sorbitol Solution, pH 4.5) exhibited severe degradation, with total impurities reaching 1.188% w/v and significant formation of Sitagliptin Acid (0.764% w/v) and Sitagliptin Styrylacetyl Analog (0.379% w/v). These findings suggest that a slightly acidic pH (3.3-3.5) with stabilizers like HCl or NaEDTA offers optimal stability for Sitagliptin, whereas higher pH values, particularly in the presence of KHCO₃ or sorbitol, lead to increased impurity formation and reduced stability.

**Table 2: Stability Data for Sitagliptin after 1 month at 40°C**

| **Batch No.** | **Additional Stabilizing Excipient** | **pH** | **Sitagliptin Assay** | **Total Impurities** | **Sitagliptin Acid** | **Impurity-B** | **Sitagliptin Phenylcrotonyl Analog** | **Sitagliptin Styrylacetyl Analog** |
|---|---|---|---|---|---|---|---|---|
| **Specification (%w/v)** | | | **90.0 - 110.0** | **NMT 0.6** | **NMT 0.2** | **NMT 0.2** | **NMT 0.2** | **NMT 0.2** |
| **75.1** | HCl | 3.3 | 99.4 | 0.058 | 0.058 | ND | ND | ND |
| **75.2** | HCl | 3.5 | 99.6 | 0.053 | 0.053 | ND | ND | ND |
| **75.3** | HCl | 3.7 | 99.1 | 0.059 | 0.059 | ND | ND | ND |
| **75.4** | HCl | 3.9 | 100.5 | 0.064 | 0.064 | ND | ND | ND |
| **75.5** | HCl/Xanthan Gum | 3.5 | 98.9 | 0.051 | 0.051 | ND | ND | ND |
| **83** | KHCO₃/H₃PO₄ | 3.5 | 99.3 | 0.067 | 0.067 | ND | ND | ND |
| **94** | KHCO₃/H₃ PO₄ | 4.5 | 100.3 | 0.125 | 0.125 | ND | ND | ND |

**Table 3: Stability Data for Sitagliptin after 2 months at 40°C**

| **Batch No.** | **Additional Stabilizing Excipient** | **pH** | **Sitagliptin Assay** | **Total Impurities** | **Sitagliptin Acid** | **Impurity-B** | **Sitagliptin Phenylcrotonyl Analog** | **Sitagliptin Styrylacetyl Analog** |
|---|---|---|---|---|---|---|---|---|
| **Specification (%w/v)** | | | **90.0 - 110.0** | **NMT 0.6** | **NMT 0.2** | **NMT 0.2** | **NMT 0.2** | **NMT 0.2** |
| **75.1** | HCl | 3.3 | 101.6 | 0.141 | 0.093 | ND | ND | ND |
| **75.2** | HCl | 3.5 | 101.6 | 0.152 | 0.088 | ND | ND | ND |
| **75.3** | HCl | 3.7 | 102.1 | 0.165 | 0.098 | ND | ND | ND |
| **75.4** | HCl | 3.9 | 103.6 | 0.171 | 0.102 | ND | ND | ND |
| **75.5** | HCl/Xanthan Gum | 3.5 | 102.3 | 0.138 | 0.091 | ND | ND | ND |
| **83** | KHCO₃/H₃ PO₄ | 3.5 | 101.5 | 0.146 | 0.102 | ND | ND | ND |

Stability data for Sitagliptin at 40°C over two months confirms that all formulations maintained assay values within the acceptable range (90.0% w/v-110.0% w/v) with minimal impurity formation. Formulations with HCl as the stabilizing excipient (pH 3.3-3.9) exhibited excellent stability, with total impurities ranging from 0.141% w/v to 0.171% w/v, well below the 0.6% w/v threshold. The addition of xanthan gum (Batch 75.5, pH 3.5) did not negatively impact stability, as it maintained low impurity levels (0.138% w/v). The KHCO₃/H₃PO₄-based formulation (Batch 83, pH 3.5) also demonstrated good stability, with slightly higher total impurities (0.102% w/v) compared to HCl-based formulations but still within the acceptable range. Importantly, no major degradation products-such as Impurity-B, Sitagliptin Phenylcrotonyl Analog, or Sitagliptin Styrylacetyl Analog-were detected in any formulation, reinforcing the stabilizing effect of a slightly acidic pH. These findings suggest that a slightly acidic environment (pH 3.3-3.9), particularly with HCl, provides optimal stability for Sitagliptin over a two-month period at 40°C.

**Table 4: Stability Data for Sitagliptin after 1 month at RT**

| **Batch No.** | **Additional Stabilizing Excipient** | **pH** | **Sitagliptin Assay** | **Total Impurities** | **Sitagliptin Acid** | **Impuri ty-B** | **Sitagliptin Phenylcrotonyl Analog** | **Sitagliptin Styrylacetyl Analog** |
|---|---|---|---|---|---|---|---|---|
| **Specification (%w/v)** | | | **90.0 - 110.0** | **NMT 0.6** | **NMT 0.2** | **NMT 0.2** | **NMT 0.2** | **NMT 0.2** |
| **75.1** | HCl | 3.3 | 99.3 | 0.034 | 0.034 | ND | ND | ND |
| **75.2** | HCl | 3.5 | 100.3 | 0.033 | 0.033 | ND | ND | ND |
| **75.3** | HCl | 3.7 | 99.9 | 0.034 | 0.034 | ND | ND | ND |
| **75.4** | HCl | 3.9 | 99.2 | 0.034 | 0.034 | ND | ND | ND |
| **75.5** | HCl/Xanthan Gum | 3.5 | 98.9 | 0.033 | 0.033 | ND | ND | ND |
| **83** | KHCO₃/H₃ PO₄ | 3.5 | 97.7 | 0.034 | 0.034 | ND | ND | ND |
| **94** | KHCO₃/H₃ PO₄ | 4.5 | 101.0 | 0.040 | 0.040 | ND | ND | ND |

**Table 5: Stability Data for Sitagliptin after 2 months at RT**

| **Bat ch No.** | **Additional Stabilizing Excipient** | **pH** | **Sitaglip tin Assay** | **Total Impu rities** | **Sitaglipt in Acid** | **Impur ity-B** | **Sitagliptin Phenylcrot onyl Analog** | **Sitagliptin Styrylacet yl Analog** |
|---|---|---|---|---|---|---|---|---|
| **Specification (%w/v)** | | | **90.0 - 110.0** | **NMT 0.6** | **NMT 0.2** | **NMT 0.2** | **NMT 0.2** | **NMT 0.2** |
| **75.1** | HCl | 3.3 | 101.4 | 0.034 | 0.034 | ND | ND | ND |
| **75.2** | HCl | 3.5 | 102.5 | 0.035 | 0.035 | ND | ND | ND |
| **75.3** | HCl | 3.7 | 101.3 | 0.041 | 0.041 | ND | ND | ND |
| **75.4** | HCl | 3.9 | 100.5 | 0.034 | 0.034 | ND | ND | ND |
| **75.5** | HCl/Xanthan Gum | 3.5 | 100.5 | 0.035 | 0.035 | ND | ND | ND |

The stability data for Sitagliptin after two months at room temperature (RT) indicates excellent stability across all tested formulations. The assay values for all Batches remained within the acceptable range (90.0% w/v-110.0% w/v), with minimal impurity formation. Formulations with HCl as the stabilizing excipient (pH 3.3-3.9) exhibited very low total impurities (0.034% w/v-0.041% w/v), suggesting that an acidic environment effectively stabilizes Sitagliptin. The addition of xanthan gum (Batch 75.5) did not impact stability, as impurity levels remained at 0.035% w/v. The KHCO₃/H₃PO₄-based formulation (Batch 83) had a slightly lower assay (97.7% w/v) but comparable impurity levels to HCl-based formulations, indicating its relative stability. Notably, Sitagliptin Phenylcrotonyl Analog, Sitagliptin Styrylacetyl Analog, and Impurity-B were not detected. Overall, these results confirm that Sitagliptin remains highly stable at room temperature, with HCl-based formulations providing optimal stability and minimal impurity formation.

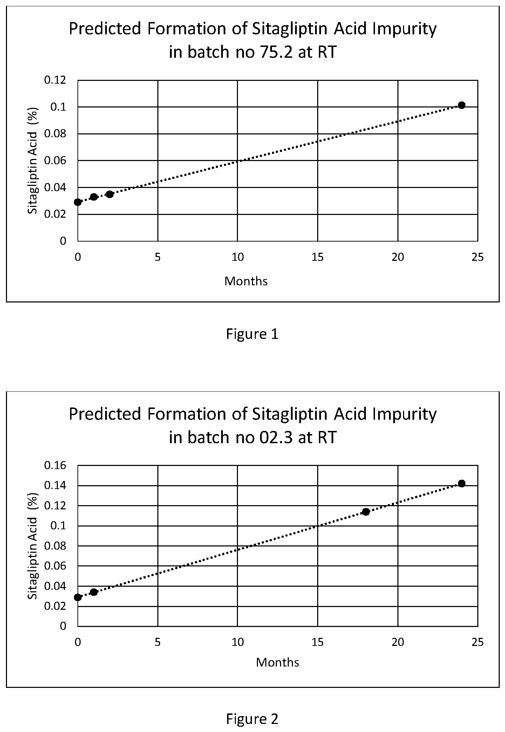

Figure 1 is a graph of Predicted Formation of Sitagliptin Acid Impurity in batch no 75.2 at RT up to 24 months, wherein:
X-Axis (Time): Represents the storage duration in months, ranging from 0 to 24 months.
Y-Axis (Impurity Level): Represents the percentage (% w/v) of Sitagliptin Acid impurity formed in the batch.
Data Characteristics of Figure 1:
The curve follows a linear trend, indicated by the dotted best-fit line.
The impurity formation appears to increase gradually over time

**Table 6: Stability Data for Sitagliptin after 18 months at RT**

| **Batch No.** | **Additional Stabilizing Excipient** | **pH** | **Sitagliptin Assay** | **Total Impurities** | **Sitagliptin Acid** | **Impurity-B** | **Sitagliptin Phenylcrotonyl Analog** | **Sitagliptin Styrylacetyl Analog** |
|---|---|---|---|---|---|---|---|---|
| **Specification (%w/v)** | | | **90.0 - 110.0** | **NMT 0.6** | **NMT 0.2** | **NMT 0.2** | **NMT 0.2** | **NMT 0.2** |
| **02.2** | KHCO₃/H₃ PO₄ | 4.5 | 100.4 | 0.127 | 0.127 | ND | ND | ND |
| **02.3** | KHCO₃/H₃ PO₄ | 3.5 | 100.0 | 0.114 | 0.114 | ND | ND | ND |

The stability data for Sitagliptin after 18 months at room temperature (RT) indicates excellent long-term stability, with assay values remaining within the acceptable range (100.0% w/v-100.4% w/v). Both formulations, using KHCO₃/H₃PO₄ as stabilizing excipients at pH 3.5 and 4.5, maintained low impurity levels. Total impurities were well below the 0.6% w/v threshold, with values of 0.127% w/v and 0.114% w/v, respectively. Sitagliptin Acid remained the only detected impurity, while Impurity-B, Sitagliptin Phenylcrotonyl Analog, and Sitagliptin Styrylacetyl Analog were not detected in either Batch. These results suggest that the KHCO₃/H₃PO₄ buffer system effectively stabilizes Sitagliptin over an extended period at room temperature, maintaining both potency and impurity levels well within acceptable limits.

The stability data for Sitagliptin across various conditions indicate that formulations with a slightly acidic pH (3.0-3.9) provide optimal stability, particularly when HCl is used as a pH modifying agent. At elevated temperatures (60°C for one week), HCl-based formulations maintained assay values within the acceptable range (90.0% w/v-110.0% w/v) with minimal impurity formation, while higher pH formulations (≥4.5), especially with glycerin and sorbitol, showed degradation. After one month at 40°C, all formulations remained stable, with HCl-based formulations exhibiting the lowest impurity levels (≤0.064% w/v). At room temperature (RT) for one month, all formulations maintained excellent stability, with total impurities as low as 0.033% w/v-0.034% w/v, further confirming that a slightly acidic environment stabilizes Sitagliptin effectively. Long-term stability data (18 months at RT) showed that KHCO₃/H₃PO₄ formulations at pH 3.5 and 4.5 remained stable, with low impurity levels (≤0.127% w/v). Collectively, these results highlight the importance of maintaining a slightly acidic pH (3.0-3.9) to optimize Sitagliptin stability, with HCl-based formulations demonstrating the most consistent performance across storage conditions.

Figure 2 is a graph of Predicted Formation of Sitagliptin Acid Impurity in batch no 02.3 at RT up to 24 months, wherein:
X-Axis (Time): Time in months, ranging from 0 to 24.
Y-Axis (Impurity Level): Percentage (% w/v) of Sitagliptin Acid impurity detected/predicted.
Trend of Figure 2: The data follows a linear progression, as shown by the dotted trend line. To improve palatability, different flavoring agents were evaluated:

**Table 7: Sensory Evaluation of Flavoring Agents**

| **Flavoring Agent** | **Sensory Evaluation** |
|---|---|
| Peppermint | Acceptable |
| Strawberry | Highly Acceptable |
| Orange | Acceptable |
| Cherry | Acceptable |

Based on sensory evaluation, strawberry flavor was identified as the preferred choice due to its superior taste profile. Importantly, its inclusion had no adverse impact on stability, ensuring both palatability and pharmaceutical robustness. However, a combination of peppermint and strawberry can also be used.

These findings provide a robust basis for the final formulation selection, ensuring product stability and patient compliance.

The composition maintains sitagliptin or its pharmaceutically acceptable salt, preferably sitagliptin phosphate, at a concentration of 25 mg per 5 mL while achieving long-term stability of at least 24 months at room temperature (25°C ± 2°C) without compromising API potency or exceeding degradation product levels of 0.6% w/v.

This enhanced stability is achieved through precise pH modulation within the range of 2.5 to 4.5 using hydrochloric acid, which minimizes hydrolytic and oxidative degradation. Additionally, the exclusion of sugar alcohols eliminates potential gastrointestinal discomfort and improve stability profile, while sucralose effectively masks bitterness without impacting stability of API. The inclusion of a preservative ensures microbial integrity, further supporting extended shelf life. Moreover, the formulation remains free from visible precipitation or turbidity over time, owing to the controlled selection of excipients and a filtration step. To mitigate light-induced degradation, the composition is stored in light-protective HDPE containers under nitrogen flushing. These advancements collectively establish a novel, commercially viable, and patient-friendly alternative to conventional oral sitagliptin formulations, addressing key barriers to oral liquid gliptin development.

These formulation optimizations collectively contribute to enhanced API stability, ensuring a robust oral liquid composition suitable for long-term storage. The following embodiments further define specific compositional aspects of the present invention.

In one of the embodiments of the present invention it provides a pharmaceutical oral solution composition comprising Sitagliptin or a pharmaceutically acceptable salt thereof at a concentration of 0.25 - 3.5% w/v, preferably 25 mg per 5 mL, wherein the composition exhibits improved stability for at least 24 months at room temperature (25°C ± 2°C) while maintaining API potency of at least 99% w/v±2% w/v and degradation product levels with the total impurities not exceeding 0.6% w/v.

In another embodiment of the present invention the composition further comprises a preservative, a sweetener, a flavoring agent, pH adjusting agent, and Purified water; optionally chelating agents, antioxidants and wetting agents, wherein the composition does not contain sugar alcohols.

In another embodiment, the present invention relates to the composition of wherein Sitagliptin phosphate is in micronized and nonmicronized form.

One of the embodiments of the present invention relates to the composition wherein the preservative is selected from methyl paraben, ethyl paraben, propyl paraben, butyl paraben, benzoic acid, sodium benzoate, benzyl alcohol, sorbic acid, potassium sorbate, and combinations thereof.

Another embodiment of the present invention relates to the composition, wherein the preservative is sodium benzoate or benzoic acid in an amount ranging from about 0.05% to about 0.2% w/v, more preferably from about 0.075% to about 0.15% w/v, and most preferably around 0.1% w/v.

In yet other embodiment the present invention relates to the composition wherein the sweetener is selected from suitable artificial non-sugar alcohol sweetening agents selected from sucralose, sodium saccharin, acesulfame-K, aspartame, alitame, cyclamate, stevioside, glycyrrhizin, neo hesperidin, dihydrochalcone, thaumatin, or combinations thereof.

In yet other embodiment the present invention relates to the composition wherein the sweetener is sucralose in a concentration of 0.1 - 2.0 % w/v, more preferably from about 0.15% to about 1.0% w/v, and most preferably from about 0.5% to about 0.7% w/v.

In yet other embodiment the present invention relates to the composition wherein the flavoring agent is selected from natural or synthetic flavours such as banana, lemon, orange, grape, lime, grapefruit, vanilla, bubble gum, peppermint, fantasy fruit masking flavour, and fruit essences including apple, banana, pear, peach, strawberry, raspberry, cherry, plum, pineapple, and apricot. Other examples include synthetic flavour oils, flavoring aromatics, or natural oils like cinnamon oil, oil of wintergreen, peppermint oil, clove oil, citrus oil, bay oil, anise oil, eucalyptus, thyme oil, cedar leaf oil, oil of nutmeg, oil of sage, oil of bitter almonds, cassia oil, maltol, ethyl vanillin, menthol, citric acid, fumaric acid, ethyl maltol, tartaric acid, or combinations thereof.

In yet other embodiment the present invention relates to the composition wherein the flavoring agent is strawberry flavor in a concentration of about 0.1% to about 2.0% w/v, preferably from about 0.3% to about 0.6% w/v, and most preferably from about 0.35% to about 0.45% w/v.

In yet other embodiment the present invention relates to the composition wherein the chelating agent is selected from disodium edentate salt (EDTA), tartaric acid, malic acid, citric acid, and combinations thereof.

In yet other embodiment the present invention relates to the composition wherein the chelating agent is disodium edentate salt in a concentration of 0.01-0.1% w/v.

In yet other embodiment the present invention relates to the composition wherein the antioxidant is selected from butylated hydroxyl anisole, sodium metabisulfite, butylated hydroxyl toluene, tocopherol, ascorbyl palmitate, ascorbic acid, sodium sulphite, sodium thiosulfate, propyl gallate, and combinations thereof.

In yet other embodiment the present invention relates to the composition wherein an antioxidant is butylated hydroxyl anisole (BHA) or metabisulphite, in a concentration of 0.01-0.10 % w/v
In yet other embodiment the present invention relates to the composition wherein the wetting agent is selected from sodium lauryl sulphate, sorbitan esters of fatty acids, sorbitan monolaurate, sorbitan monooleate, sorbitan trioleate, sorbitan monopalmitate, sorbitan monostearate, sorbitan tristearate, ethylene oxide-propylene oxide block copolymers, lecithins, oleic acid and oleic acid salts, propylene glycol monostearate and monolaurate, glycerol monostearate and monooleate, fatty alcohol-polyethylene glycol ethers, fatty acid-polyethylene glycol esters, sodium dodecyl sulphate, dioctyl sodium sulphosuccinate, ethoxylated mono- and diglycerides, sucrose fatty acid esters, fatty acid salts, ethoxylated triglycerides, polyoxyethylated hydrogenated castor oil, sterol, and combinations thereof, the wetting agent preferably comprising sorbitan monooleate, more preferably polysorbate 80.

In yet other embodiment the present invention relates to the composition wherein the wetting agent is polysorbate 80, in a concentration of 0.01 - 0.5% w/v.

In yet other embodiment the present invention relates to the composition wherein the pH-adjusting agent is selected from hydrochloric acid, nitric acid, phosphoric acid, sulphuric acid, acetic acid, citric acid, malic acid, lactic acid, propionic acid, tartaric acid, ammonia solutions, monoethanolamine, diethanol-amine, triethanolamine, meglumine, sodium acetate, sodium citrate, potassium bicarbonate, potassium citrate, potassium hydroxide, sodium bicarbonate, sodium borate, sodium hydroxide or combinations thereof.

In yet other embodiment the present invention relates to the composition wherein the pH-adjusting agent is hydrochloric acid.

In yet other embodiment the present invention relates to the composition wherein the pH is maintained in the range of 2.0 to 5.0 for optimal stability.

In yet other embodiment the present invention relates to the composition wherein it comprises one or more additional antidiabetic drugs, which are preferably selected from acarbose, miglitol, repaglinide, nateglinide, glibenclamide, gliclazide, glimepiride, glipizide, tolbutamide, metformin, phenformin, rosiglitazone, pioglitazone, troglitazone, farglitazar, englitazone, darglitazone, isaglitazone, reglitazar, rivoglitazone, liraglutide, muraglitazar, peliglitazar, tesaglitazar, canagliflozin, dapagliflozin, remogliflozin, sergliflozin, and their pharmaceutically acceptable salts or esters. Furthermore, the additional antidiabetic drug may include a DPP-IV inhibitor.

In yet other embodiment the present invention relates to the composition wherein the solution remains free from visible precipitation or turbidity over the storage period.

In yet other embodiment the present invention relates to the composition wherein the solution is stored in amber glass bottles, opaque plastic containers, or high-density polyethylene (HDPE) bottles to minimize API degradation.

In yet other embodiment the present invention provides a stable pharmaceutical oral solution composition comprising Sitagliptin or a pharmaceutically acceptable salt thereof, wherein the composition maintains controlled levels of impurities and degradation products within specified limits to ensure stability and efficacy. Specifically, the composition contains Sitagliptin Acid not more than (NMT) 0.2% w/v, Sitagliptin Triazecine Analog NMT 0.2%w/v, Sitagliptin Styrylacetyl Analog NMT 0.2% w/v, Sitagliptin Phenyl crotonyl Analog NMT 0.2%w/v, and any unspecified degradation product NMT 0.2%w/v. The formulation is designed with pharmaceutically acceptable excipients, including stabilizers, antioxidants, pH adjusters, and chelating agents, to prevent degradation and maintain impurity levels within the specified limits throughout its shelf life, thereby ensuring the safety, efficacy, and stability of the composition.

In one embodiment of the present invention, the formation of Sitagliptin Acid as a degradation impurity was monitored and predicted over time for batch numbers 75.2 and 02.3.

stored at room temperature (RT). The predicted increase in impurity content followed a substantially linear trend, with Sitagliptin Acid content rising from approximately 0.03% w/v at time zero to up to 0.14% w/v at 24 months. This data supports the stability profile of the compound and assists in setting appropriate shelf-life and impurity limits for regulatory submissions.

The above embodiment confirms the utility of the claimed formulation in maintaining acceptable impurity levels of Sitagliptin Acid, within regulatory limits, over extended periods under ambient storage conditions.

In yet other embodiment the present invention relates to a method for preparing a stable pharmaceutical oral solution composition comprising Sitagliptin salt, the method comprising:
- Dissolving preservative, sweetener, and flavoring agent in purified water under continuous stirring,
- Admixing, if present, the wetting agent,
- Admixing, the pH agent or a portion thereof,
- Admixing, Sitagliptin salt under controlled temperature (20-35° C),
- optionally, homogenising the obtained mixture, ensuring uniform dissolution and homogeneity,
- If necessary, adjust the pH to the envisaged value by addition of pH adjusting agent,
- If necessary, adjust the final volume by adding from the rest of the water,
- Optionally, filter through 1 to 10 µm filter, and
- Filling the solution into appropriate container.

In yet other embodiment the present invention relates to the method wherein the process is conducted at a temperature between 15°C and 25°C.

In yet other embodiment the present invention relates to the method wherein nitrogen flushing is performed before sealing the bottles to enhance the stability of the composition by reducing oxidative degradation.

In yet other embodiment the present invention relates to the method wherein the solution is filtered through 1-10 micron filter, preferably a 0.2-micron filter to remove particulate matter.

In yet other embodiment the present invention relates to the method wherein the final product is stored in amber glass bottles, opaque plastic containers to protect against light-induced degradation.

In yet other embodiment the present invention the container is HDPE bottle.

In yet other embodiment the present invention relates to the method wherein the formulation is tested for chemical stability by High-Performance Liquid Chromatography (HPLC) with a detection limit of 0.025% w/v. and quantitation limit of 0.05% w/v.

In yet other embodiment the present invention relates to the method wherein no sterile filtration is required.

These embodiments illustrate the novel formulation approach, balancing stability, patient compliance, and commercial viability to overcome traditional challenges in oral liquid sitagliptin development.

The oral pharmaceutical composition of the present invention comprises Sitagliptin in an amount ranging from about 0.25% to about 3.5% w/v, preferably 25mg/5mL. The composition further includes a preservative such as sodium benzoate or benzoic acid in an amount ranging from about 0.05% to about 0.2% w/v, more preferably from about 0.075% to about 0.15% w/v, and most preferably around 0.1% w/v. A sweetener such as sucralose is incorporated at a concentration ranging from about 0.1% to about 2.0% w/v, more preferably from about 0.15% to about 1.0% w/v, and most preferably from about 0.5% to about 0.7% w/v. The flavoring agent, e.g., a strawberry flavor, is present in an amount of about 0.1% to about 2.0% w/v, preferably from about 0.3% to about 0.6% w/v, and most preferably from about 0.35% to about 0.45% w/v. Where applicable, a buffering or stabilizing agent such as potassium bicarbonate may be included at about 0.01% to 0.5% w/v, preferably from about 0.1% to 0.3% w/v, and most preferably from about 0.2% to 0.3% w/v. In one embodiment, a viscosity-enhancing agent such as xanthan gum may be included in an amount ranging from 0.01% to 0.2% w/v, preferably from 0.05% to 0.1% w/v, and most preferably around 0.01% to 0.02% w/v. The pH of the formulation is adjusted using a pharmaceutically acceptable acid such as hydrochloric acid, phosphoric acid, citric acid, or malic acid to a value within the range of 2.0 to 5.0, preferably from about 2.5 to 4.5, and most preferably from about 3.3 to 3.7. The composition is brought to final volume with purified water.

The above embodiments are further supported by the following Examples 1-16.

### Example 1: Sitagliptin Oral Solution, 25 mg/5 mL (Batch no. 75.1)

| **Ingredient** | **Formula Qty. (g/100 mL)** | **Range (%w/v)** |
|---|---|---|
| Sitagliptin free Base (as Sitagliptin Phosphate, USP) | 0.50 | 0.25 - 3.5 |
| Sodium Benzoate, NF | 0.1 | 0.05 - 0.2 |
| Sucralose, NF | 0.7 | 0.1 - 2.0 |
| Strawberry Flavor, Natural WONF 915.09640 | 0.4 | 0.1 - 2.0 |
| Hydrochloric Acid, NF | To adjust pH to 3.3 | To adjust pH to 2.5 - 4.5 |
| Purified Water, USP | Q.S. to 100 mL | Q.S. to 100 mL |

### Example 2: Sitagliptin Oral Solution, 25 mg/5 mL (Batch no. 75.2)

| **Ingredient** | **Formula Qty. (g/100 mL)** | **Range (%w/v)** |
|---|---|---|
| Sitagliptin free Base (as Sitagliptin Phosphate, USP) | 0.50 | 0.25 - 3.5 |
| Sodium Benzoate, NF | 0.1 | 0.05 - 0.2 |
| Sucralose, NF | 0.7 | 0.1 - 2.0 |
| Strawberry Flavor, Natural WONF 915.09640 | 0.4 | 0.1 - 2.0 |
| Hydrochloric Acid, NF | To adjust pH to 3.5 | To adjust pH to 2.5 - 4.5 |
| Purified Water, USP | Q.S. to 100 mL | Q.S. to 100 mL |

### Example 3: Sitagliptin Oral Solution, 25 mg/5 mL (Batch no. 75.3)

| **Ingredient** | **Formula Qty. (g/100 mL)** | **Range (%w/v)** |
|---|---|---|
| Sitagliptin free Base (as Sitagliptin Phosphate, USP) | 0.50 | 0.25 - 3.5 |
| Sodium Benzoate, NF | 0.1 | 0.05 - 0.2 |
| Sucralose, NF | 0.7 | 0.1 - 2.0 |
| Strawberry Flavor, Natural WONF 915.09640 | 0.4 | 0.1 - 2.0 |
| Hydrochloric Acid, NF | To adjust pH to 3.7 | To adjust pH to 2.5 - 4.5 |
| Purified Water, USP | Q.S. to 100 mL | Q.S. to 100 mL |

### Example 4: Sitagliptin Oral Solution, 25 mg/5 mL (Batch no. 75.4)

| **Ingredient** | **Formula Qty. (g/100 mL)** | **Range (%w/v)** |
|---|---|---|
| Sitagliptin free Base (as Sitagliptin Phosphate, USP) | 0.50 | 0.25 - 3.5 |
| Sodium Benzoate, NF | 0.1 | 0.05 - 0.2 |
| Sucralose, NF | 0.7 | 0.1 - 2.0 |
| Strawberry Flavor, Natural WONF 915.09640 | 0.4 | 0.1 - 2.0 |
| Hydrochloric Acid, NF | To adjust pH to 3.9 | To adjust pH to 2.5 - 4.5 |
| Purified Water, USP | Q.S. to 100 mL | Q.S. to 100 mL |

### Example 5: Sitagliptin Oral Solution, 25 mg/5 mL (Batch no. 75.5)

| **Ingredient** | **Formula Qty. (g/100 mL)** | **Range (%w/v)** |
|---|---|---|
| Sitagliptin free Base (as Sitagliptin Phosphate, USP) | 0.50 | 0.25 - 3.5 |
| Sodium Benzoate, NF | 0.1 | 0.05 - 0.2 |
| Sucralose, NF | 0.7 | 0.1 - 2.0 |
| Strawberry Flavor, Natural WONF 915.09640 | 0.4 | 0.1 - 2.0 |
| Xanthan Gum, NF | 0.01 | 0.01 - 0.2 |
| Hydrochloric Acid, NF | To adjust pH to 3.5 | To adjust pH to 2.5 - 4.5 |
| Purified Water, USP | Q.S. to 100 mL | Q.S. to 100 mL |

### Example 6: Sitagliptin Oral Solution, 25 mg/5 mL (Batch no. 83 or Batch no. 02.3)

| **Ingredient** | **Formula Qty. (g/100 mL)** | **Range (%w/v)** |
|---|---|---|
| Sitagliptin free Base (as Sitagliptin Phosphate, USP) | 0.50 | 0.25 - 3.5 |
| Benzoic Acid, NF | 0.1 | 0.05 - 0.2 |
| Sucralose, NF | 0.15 | 0.1 - 2.0 |
| Strawberry Flavor, Natural WONF 915.09640 | 0.3 | 0.1 - 2.0 |
| KHCO₃, USP | 0.25 | 0.01 - 0.5 |
| H₃PO₄, NF | To adjust pH to 3.5 | To adjust pH to 2.5 - 4.5 |
| Purified Water, USP | Q.S. to 100 mL | Q.S. to 100 mL |

### Example 7: Sitagliptin Oral Solution, 25 mg/5 mL (Batch no. 94 or Batch no. 02.2)

| **Ingredient** | **Formula Qty. (g/100 mL)** | **Range (%w/v)** |
|---|---|---|
| Sitagliptin free Base (as Sitagliptin Phosphate, USP) | 0.50 | 0.25 - 3.5 |
| Benzoic Acid, NF | 0.1 | 0.05 - 0.2 |
| Sucralose, NF | 0.15 | 0.1 - 2.0 |
| Strawberry Flavor, Natural WONF 915.09640 | 0.3 | 0.1 - 2.0 |
| KHCO₃, USP | 0.25 | 0.01 -0.5 |
| H₃PO₄, NF | To adjust pH to 4.5 | To adjust pH to 2.5 - 4.5 |
| Purified Water, USP | Q.S. to 100 mL | Q.S. to 100 mL |

### Example 8: Sitagliptin Oral Solution, 25 mg/5 mL (Batch no. 138.1)

| **Ingredient** | **Formula Qty. (g/100 mL)** | **Range (%w/v)** |
|---|---|---|
| Sitagliptin free Base (as Sitagliptin Phosphate, USP) | 0.50 | 0.25 - 3.5 |
| Sodium Benzoate, NF | 0.1 | 0.05 - 0.2 |
| Sucralose, NF | 0.7 | 0.1 - 2.0 |
| Strawberry Flavor, Natural WONF 915.09640 | 0.4 | 0.1 - 2.0 |
| KHCO₃, USP | 0.25 | 0.01 - 0.5 |
| Hydrochloric Acid, NF | To adjust pH to 3.5 | To adjust pH to 2.5 - 4.5 |
| Purified Water, USP | Q.S. to 100 mL | Q.S. to 100 mL |

### Example 9: Sitagliptin Oral Solution, 25 mg/5 mL (Batch no. 138.2)

| **Ingredient** | **Formula Qty. (g/100 mL)** | **Range (%w/v)** |
|---|---|---|
| Sitagliptin free Base (as Sitagliptin Phosphate, USP) | 0.50 | 0.25 - 3.5 |
| Sodium Benzoate, NF | 0.1 | 0.05 - 0.2 |
| Sucralose, NF | 0.7 | 0.1 - 2.0 |
| Strawberry Flavor, Natural WONF 915.09640 | 0.4 | 0.1 - 2.0 |
| Phosphoric Acid, NF | To adjust pH to 3.5 | To adjust pH to 2.5 - 4.5 |
| Purified Water, USP | Q.S. to 100 mL | Q.S. to 100 mL |

### Example 10: Sitagliptin Oral Solution, 25 mg/5 mL (Batch no. 138.3)

| **Ingredient** | **Formula Qty. (g/100 mL)** | **Range (%w/v)** |
|---|---|---|
| Sitagliptin free Base (as Sitagliptin Phosphate, USP) | 0.50 | 0.25 - 3.5 |
| Sodium Benzoate, NF | 0.1 | 0.05 - 0.2 |
| Sucralose, NF | 0.7 | 0.1 - 2.0 |
| Strawberry Flavor, Natural WONF 915.09640 | 0.4 | 0.1 - 2.0 |
| Citric Acid, NF | To adjust pH to 3.5 | To adjust pH to 2.5 - 4.5 |
| Purified Water, USP | Q.S. to 100 mL | Q.S. to 100 mL |

### Example 11: Sitagliptin Oral Solution, 25 mg/5 mL (Batch no. 138.4)

| **Ingredient** | **Formula Qty. (g/100 mL)** | **Range (%w/v)** |
|---|---|---|
| Sitagliptin free Base (as Sitagliptin Phosphate, USP) | 0.50 | 0.25 - 3.5 |
| Sodium Benzoate, NF | 0.1 | 0.05 - 0.2 |
| Sucralose, NF | 0.7 | 0.1 - 2.0 |
| Strawberry Flavor, Natural WONF 915.09640 | 0.4 | 0.1 - 2.0 |
| Malic Acid, NF | To adjust pH to 3.5 | To adjust pH to 2.5 - 4.5 |
| Purified Water, USP | Q.S. to 100 mL | Q.S. to 100 mL |

### Example 12: Sitagliptin Oral Solution, 25 mg/5 mL (Batch no. 138.5)

| **Ingredient** | **Formula Qty. (g/100 mL)** | **Range (%w/v)** |
|---|---|---|
| Sitagliptin free Base (as Sitagliptin Phosphate, USP) | 0.50 | 0.25 - 3.5 |
| Sodium Benzoate, NF | 0.1 | 0.05 - 0.2 |
| Sucralose, NF | 0.7 | 0.1 - 2.0 |
| Strawberry Flavor, Natural WONF 915.09640 | 0.4 | 0.1 - 2.0 |
| Hydrochloric Acid, NF | To adjust pH to 4.5 | To adjust pH to 2.5 - 4.5 |
| Purified Water, USP | Q.S. to 100 mL | Q.S. to 100 mL |

### Example 13: Sitagliptin Oral Solution, 25 mg/5 mL (Batch no. 138.6)

| **Ingredient** | **Formula Qty. (g/100 mL)** | **Range (%w/v)** |
|---|---|---|
| Sitagliptin free Base (as Sitagliptin Phosphate, USP) | 0.50 | 0.25 - 3.5 |
| Sodium Benzoate, NF | 0.1 | 0.05 - 0.2 |
| Sucralose, NF | 0.7 | 0.1 - 2.0 |
| Edetate Disodium, USP Dihydrate | 0.1 | 0.01 - 0.2 |
| Strawberry Flavor, Natural WONF 915.09640 | 0.4 | 0.1 - 2.0 |
| Hydrochloric Acid, NF | To adjust pH to 3.5 | To adjust pH to 2.5 - 4.5 |
| Purified Water, USP | Q.S. to 100 mL | Q.S. to 100 mL |

### Example 14: Sitagliptin Oral Solution, 25 mg/5 mL (Batch no. 138.7)

| **Ingredient** | **Formula Qty. (g/100 mL)** | **Range (%w/v)** |
|---|---|---|
| Sitagliptin free Base (as Sitagliptin Phosphate, USP) | 0.50 | 0.25 - 3.5 |
| Sodium Benzoate, NF | 0.1 | 0.05 - 0.2 |
| Sucralose, NF | 0.7 | 0.1 - 2.0 |
| Glycerin, USP | 10.0 | 5.0 - 20.0 |
| Strawberry Flavor, Natural WONF 915.09640 | 0.4 | 0.1 - 2.0 |
| Hydrochloric Acid, NF | To adjust pH to 3.5 | To adjust pH to 2.5 - 4.5 |
| Purified Water, USP | Q.S. to 100 mL | Q.S. to 100 mL |

### Example 15: Sitagliptin Oral Solution, 25 mg/5 mL (Batch no. 138.8)

| **Ingredient** | **Formula Qty. (g/100 mL)** | **Range (%w/v)** |
|---|---|---|
| Sitagliptin free Base (as Sitagliptin Phosphate, USP) | 0.50 | 0.25 - 3.5 |
| Sodium Benzoate, NF | 0.1 | 0.05 - 0.2 |
| Sucralose, NF | 0.7 | 0.1 - 2.0 |
| Sorbitol Solution 70%, USP | 10.0 | 5.0 - 20.0 |
| Strawberry Flavor, Natural WONF 915.09640 | 0.4 | 0.1 - 2.0 |
| Hydrochloric Acid, NF | To adjust pH to 3.5 | To adjust pH to 2.5 - 4.5 |
| Purified Water, USP | Q.S. to 100 mL | Q.S. to 100 mL |

### Example 16: Sitagliptin Oral Solution, 25 mg/5 mL (Batch no. 138.9)

| **Ingredient** | **Formula Qty. (g/100 mL)** | **Range (%w/v)** |
|---|---|---|
| Sitagliptin free Base (as Sitagliptin Phosphate, USP) | 0.50 | 0.25 - 3.5 |
| Sodium Benzoate, NF | 0.1 | 0.05 - 0.2 |
| Sucralose, NF | 0.7 | 0.1 - 2.0 |
| Sorbitol Solution 70%, USP | 10.0 | 5.0 - 20.0 |
| Strawberry Flavor, Natural WONF 915.09640 | 0.4 | 0.1 - 2.0 |
| Hydrochloric Acid, NF | To adjust pH to 4.5 | To adjust pH to 2.5 - 4.5 |
| Purified Water, USP | Q.S. to 100 mL | Q.S. to 100 mL |

### Method of Preparation

1. Dissolving sodium benzoate in purified water with agitation (200 - 500 rpm) at 15 - 25°C, followed by the addition of sweeteners and flavoring agents.
2. Adjusting the pH of the mixture to about 3.3 - 3.7 using hydrochloric acid.
3. Adding Sitagliptin phosphate and mixing until dissolved.
4. Further adjusting the pH of the mixture to between about 3.3 - 3.6 using hydrochloric acid.
5. quantum satis/sufficient quantity (QS) to the final volume.

While the invention has been described in terms of its specific embodiments, certain modifications and equivalents will be apparent to those skilled in the art and are intended to be included within the scope of the invention. To further illustrate the invention, the following detailed description outlines exemplary embodiments, including preferred compositions, methods of preparation, and therapeutic applications.

A pharmaceutical oral solution composition as described herein, comprising Sitagliptin or a pharmaceutically acceptable salt thereof at a concentration of about 0.25% to about 3.5% w/v, wherein the composition exhibits improved stability for at least 24 months at room temperature (25°C ± 2°C), maintains an active pharmaceutical ingredient (API) potency of at least 99% ± 2% w/v, and comprises total degradation product levels not exceeding 0.6% w/v, and wherein the pH of the composition is in the range of about 2.0 to about 5.0.

The pharmaceutical oral solution composition as described herein may further comprise a preservative, a sweetener, a flavoring agent, optionally one or more chelating agents, antioxidants, and wetting agents, a pH-adjusting agent, and purified water, wherein the composition is free from sugar alcohols.

In the pharmaceutical composition as described herein, Sitagliptin may be present in the form of a pharmaceutically acceptable salt, preferably Sitagliptin phosphate or Sitagliptin chloride.

In the pharmaceutical composition as described herein, the preservative may be selected from methyl paraben, ethyl paraben, propyl paraben, butyl paraben, benzoic acid, sodium benzoate, benzyl alcohol, sorbic acid, potassium sorbate, or combinations thereof; more preferably, the preservative is sodium benzoate at a concentration of about 0.05% to about 0.2% w/v.

In the pharmaceutical composition as described herein, the sweetener may be selected from sucralose, sodium saccharin, acesulfame-K, aspartame, alitame, cyclamate, stevioside, glycyrrhizin, neohesperidin dihydrochalcone, thaumatin, or combinations thereof; more preferably, the sweetener is sucralose at a concentration of about 0.1% to about 2.0% w/v, even more preferably from about 0.15% to about 1.0% w/v, and most preferably from about 0.5% to about 0.7% w/v.

In the pharmaceutical composition as described herein, the flavoring agent may be selected from natural or synthetic flavors, including but not limited to banana, lemon, orange, grape, lime, grapefruit, vanilla, bubble gum, peppermint, fruit essences (apple, banana, pear, peach, strawberry, raspberry, cherry, plum, pineapple, apricot), synthetic flavor oils, and natural oils (cinnamon oil, oil of wintergreen, peppermint oil, clove oil, citrus oil, bay oil, anise oil, eucalyptus oil, thyme oil, cedar leaf oil, nutmeg oil, sage oil, bitter almond oil, cassia oil), or combinations thereof; more preferably, the flavoring agent is strawberry flavor at a concentration of about 0.1% to about 2.0% w/v, preferably from about 0.3% to about 0.6% w/v, and most preferably from about 0.35% to about 0.45% w/v.

In the pharmaceutical composition as described herein, the chelating agent may be selected from disodium edetate (EDTA), tartaric acid, malic acid, citric acid, or combinations thereof.

In the pharmaceutical composition as described herein, the antioxidant may be selected from butylated hydroxyanisole (BHA), sodium metabisulfite, butylated hydroxytoluene (BHT), tocopherol, ascorbyl palmitate, ascorbic acid, sodium sulfite, sodium thiosulfate, propyl gallate, or combinations thereof; preferably, the antioxidant is BHA or sodium metabisulfite.

In the pharmaceutical composition as described herein, the wetting agent may be selected from sodium lauryl sulfate, sorbitan esters, polysorbates, lecithins, oleic acid and salts thereof, propylene glycol monostearate, glycerol monostearate, ethoxylated mono- and diglycerides, sucrose fatty acid esters, ethoxylated triglycerides, polyoxyethylated hydrogenated castor oil, sterols, or combinations thereof; more preferably, the wetting agent is polysorbate 80 at a concentration of about 0.01% to about 0.5% w/v.

In the pharmaceutical composition as described herein, the pH-adjusting agent may be selected from hydrochloric acid, nitric acid, phosphoric acid, sulfuric acid, acetic acid, citric acid, malic acid, lactic acid, propionic acid, tartaric acid, ammonia solutions, monoethanolamine, diethanolamine, triethanolamine, meglumine, sodium acetate, sodium citrate, potassium bicarbonate, potassium citrate, potassium hydroxide, sodium bicarbonate, sodium borate, sodium hydroxide, or combinations thereof; and preferably, the pH-adjusting agent is hydrochloric acid.

In the pharmaceutical composition as described herein, the pH is preferably maintained between about 2.5 to about 4.5, and most preferably from about 3.3 to about 3.7 to ensure optimal stability.

In the pharmaceutical composition as described herein, the solution may be stored in a light-protective container to minimize degradation of the API, and remains free from visible precipitation or turbidity throughout the storage period.

A packaged product comprising the pharmaceutical oral solution composition as described herein, stored in a light-protective container, wherein the packaged product exhibits the stability characteristics described herein.

A method for preparing a stable pharmaceutical oral solution composition as described herein, the method comprising dissolving the preservative, sweetener, and flavoring agent in purified water under continuous stirring; optionally adding the wetting agent; adding the pH-adjusting agent or a portion thereof; adding Sitagliptin phosphate under controlled temperature conditions of about 20°C to about 35°C; optionally homogenizing the resulting mixture to ensure uniform dissolution; adjusting the pH to the target value if necessary; adjusting the final volume with purified water; optionally filtering the solution through a filter having a pore size of about 1 to 10 µm; and filling the solution into suitable containers.

In the method as described herein, the preparation may be conducted at a temperature between about 15°C and about 25°C.

In the method as described herein, the solution may be filtered through a filter of about 1 to 10 microns to remove particulate matter.

In the method as described herein, the solution may be filled into light-protective containers under nitrogen flushing.

In the method as described herein, the final product may be stored in amber glass bottles or opaque plastic containers, such as high-density polyethylene (HDPE) bottles, to protect against light-induced degradation.

In the method as described herein, the formulation may be tested for chemical stability using high-performance liquid chromatography (HPLC), wherein the method has a detection limit of about 0.025% w/v and a quantitation limit of about 0.05% w/v.

A method for treating type 2 diabetes mellitus in a subject in need thereof, comprising orally administering a therapeutically effective amount of the pharmaceutical oral solution composition as described herein.

The following additional enumerated embodiments are likewise exemplary and nonlimiting in nature.

In a first embodiment, a pharmaceutical oral solution composition comprises Sitagliptin or a pharmaceutically acceptable salt thereof at a concentration 0.25 - 3.5% w/v, wherein the composition exhibits improved stability for at least 24 months at room temperature (25°C ± 2°C), maintaining an API potency of at least 99±2% w/v and total degradation product levels not exceeding 0.6% w/v and wherein the pH of the aqueous liquid oral composition is in the range of about 2.0 to about 5.0.

A second embodiment can include the composition of the first embodiment, further comprising a preservative, a sweetener, a flavoring agent, optionally, one or more chelating agents, antioxidants, and wetting agents, a pH-adjusting agent, and purified water, wherein the composition is free from sugar alcohols.

A third embodiment can include the composition of the first or second embodiment, wherein Sitagliptin is in the form of a pharmaceutically acceptable salt, preferably Sitagliptin phosphate or Sitagliptin chloride.

A fourth embodiment can include the composition of any of the first through third embodiments, wherein the preservative is selected from methyl paraben, ethyl paraben, propyl paraben, butyl paraben, benzoic acid, sodium benzoate, benzyl alcohol, sorbic acid, potassium sorbate, or combinations thereof.

A fifth embodiment can include the composition of any of the first through fourth embodiments, wherein the preservative is sodium benzoate at a concentration of 0.05 - 0.2% w/v.

A sixth embodiment can include the composition of any of the first through fifth embodiments, wherein the sweetener is selected from sucralose, sodium saccharin, acesulfame-K, aspartame, alitame, cyclamate, stevioside, glycyrrhizin, neo hesperidin dihydrochalcone, thaumatin, or combinations thereof.

A seventh embodiment can include the composition of any of the first through sixth embodiments, wherein the sweetener is sucralose at a concentration of 0.1 - 2.0% w/v.

An eighth embodiment can include the composition of any of the first through seventh embodiments, wherein the flavoring agent is selected from natural or synthetic flavors, including but not limited to banana, lemon, orange, grape, lime, grapefruit, vanilla, bubble gum, peppermint, fruit essences (apple, banana, pear, peach, strawberry, raspberry, cherry, plum, pineapple, apricot), synthetic flavor oils, and natural oils (cinnamon oil, oil of wintergreen, peppermint oil, clove oil, citrus oil, bay oil, anise oil, eucalyptus oil, thyme oil, cedar leaf oil, nutmeg oil, sage oil, bitter almond oil, cassia oil), or combinations thereof.

A ninth embodiment can include the composition of any of the first through eighth embodiments, wherein the flavoring agent is strawberry flavor at about 0.1% to about 2.0% w/v.

A tenth embodiment can include the composition of any of the first through ninth embodiments, wherein the chelating agent is selected from disodium edetate (EDTA), tartaric acid, malic acid, citric acid, or combinations thereof.

An eleventh embodiment can include the composition of any of the first through tenth embodiments, wherein the chelating agent is selected from disodium edentate salt (EDTA), tartaric acid, malic acid, citric acid, and combinations thereof.

A twelfth embodiment can include the composition of any of the first through eleventh embodiments, wherein the antioxidant is selected from butylated hydroxyanisole (BHA), sodium metabisulfite, butylated hydroxytoluene (BHT), tocopherol, ascorbyl palmitate, ascorbic acid, sodium sulfite, sodium thiosulfate, propyl gallate, or combinations thereof.

A thirteenth embodiment can include the composition of any of the first through twelfth embodiments, wherein the antioxidant is BHA or sodium metabisulfite.

A fourteenth embodiment can include the composition of any of the first through thirteenth embodiments, wherein the wetting agent is selected from sodium lauryl sulfate, sorbitan esters, polysorbates, lecithins, oleic acid and salts thereof, propylene glycol monostearate, glycerol monostearate, ethoxylated mono- and diglycerides, sucrose fatty acid esters, ethoxylated triglycerides, polyoxyethylated hydrogenated castor oil, sterols, or combinations thereof.

A fifteenth embodiment can include the composition of any of the first through fourteenth embodiments, wherein the wetting agent is polysorbate 80 at a concentration of 0.01 - 0.5% w/v.

A sixteenth embodiment can include the composition of any of the first through fifteenth embodiments, wherein the pH-adjusting agent is selected from hydrochloric acid, nitric acid, phosphoric acid, sulfuric acid, acetic acid, citric acid, malic acid, lactic acid, propionic acid, tartaric acid, ammonia solutions, monoethanolamine, diethanolamine, triethanolamine, meglumine, sodium acetate, sodium citrate, potassium bicarbonate, potassium citrate, potassium hydroxide, sodium bicarbonate, sodium borate, sodium hydroxide, or combinations thereof.

A seventeenth embodiment can include the composition of any of the first through sixteenth embodiments, wherein the pH-adjusting agent is hydrochloric acid.

An eighteenth embodiment can include the composition of any of the first through seventeenth embodiments, wherein the pH is maintained at more preferably between 2.5-4.5 for optimal stability.

A nineteenth embodiment can include the composition of any of the first through eighteenth embodiments, wherein the solution is stored in a light-protective container to minimize API degradation.

A twentieth embodiment can include the composition of any of the first through nineteenth embodiments, wherein the formulation remains free from visible precipitation or turbidity over the storage period.

A twenty-first embodiment can include a method for preparing a stable pharmaceutical oral solution composition comprising Sitagliptin phosphate according to any of the first through twentieth embodiments, the method comprising dissolving the preservative, sweetener, and flavoring agent in purified water under continuous stirring, optionally, adding the wetting agent, adding the pH-adjusting agent or a portion thereof, adding Sitagliptin phosphate under controlled temperature (20-35°C), optionally, homogenizing the obtained mixture to ensure uniform dissolution, adjusting the pH to the target value, if necessary, adjusting the final volume with purified water, optionally, filtering the solution through a 1-10 µm pore filter, and filling the solution into appropriate containers.

A twenty-second embodiment can include the method of the twenty-first embodiment, wherein the process is conducted at a temperature between 15°C and 25°C.

A twenty-third embodiment can include the method of the twenty-first or twenty-second embodiments, wherein the solution is filtered through a 1-10 micron filter to remove particulate matter.

A twenty-fourth embodiment can include the method of any of the twenty-first through twenty-third embodiments, wherein the solution is filled into light-protective containers under nitrogen flushing.

A twenty-fifth embodiment can include the method of any of the twenty-first through twenty-fourth embodiments, wherein the final product is stored in amber glass bottles or opaque plastic containers to protect against light-induced degradation.

A twenty-sixth embodiment can include the method of any of the twenty-first through twenty-fifth embodiments, wherein the container is a high-density polyethylene (HDPE) bottle.

A twenty-seventh embodiment can include the method of any of the twenty-first through twenty-sixth embodiments, wherein the formulation is tested for chemical stability using High-Performance Liquid Chromatography (HPLC) with a detection limit of 0.025% w/v and a quantitation limit of 0.05% w/v.

A twenty-eighth embodiment can include a method of treating type 2 diabetes mellitus in a subject in need thereof, comprising orally administering a therapeutically effective amount of the pharmaceutical oral solution composition of any of the first through twentieth embodiments.

A twenty-ninth embodiment can include a packaged product comprising the pharmaceutical oral solution composition of any of the first through twentieth embodiments, stored in a light-protective container, wherein the packaged product exhibits the stability characteristics described herein.

The foregoing description of specific embodiments is intended to be illustrative and not limiting. Various modifications and alternatives can be devised by those skilled in the art without departing from the spirit and scope of the invention. Accordingly, the scope of the invention should be determined by the following claims, which are intended to cover all such modifications, equivalents, and variations.

## Claims

1. A pharmaceutical oral solution composition comprising:
(a) sitagliptin or a pharmaceutically acceptable salt thereof at a concentration of 0.25-3.5% w/v;
(b) wherein the composition exhibits improved stability for at least 24 months at room temperature (25°C ± 2°C), maintaining an API potency of at least 99 ± 2% w/v and total degradation product levels not exceeding 0.6% w/v; and
(c) wherein the pH of the aqueous liquid oral composition is in the range of about 2.0 to about 5.0.

2. The composition of claim 1, further comprising:
(a) a preservative;
(b) a sweetener;
(c) a flavoring agent;
(d) optionally, one or more chelating agents, antioxidants, and wetting agents;
(e) a pH-adjusting agent; and
(f) purified water,
wherein the composition is free from sugar alcohols.

3. The composition of claim 1 or 2, wherein the sitagliptin is in the form of a pharmaceutically acceptable salt, preferably sitagliptin phosphate or sitagliptin chloride.

4. The composition of claim 2 or 3, wherein the preservative is selected from methyl paraben, ethyl paraben, propyl paraben, butyl paraben, benzoic acid, sodium benzoate, benzyl alcohol, sorbic acid, potassium sorbate, or combinations thereof, preferably sodium benzoate at a concentration ranging from about 0.05% to about 0.2% w/v.

5. The composition of claim 2, 3 or 4 wherein the sweetener is selected from sucralose, sodium saccharin, acesulfame-K, aspartame, alitame, cyclamate, stevioside, glycyrrhizin, neohesperidin dihydrochalcone, thaumatin, or combinations thereof, preferably sucralose at a concentration of 0.1-2.0% w/v.

6. The composition of any one of claims 2 to 5, wherein the flavoring agent is selected from natural or synthetic flavors, including banana, lemon, orange, grape, lime, grapefruit, vanilla, bubble gum, peppermint, fruit essences (apple, banana, pear, peach, strawberry, raspberry, cherry, plum, pineapple, apricot), synthetic flavor oils, and natural oils (cinnamon oil, oil of wintergreen, peppermint oil, clove oil, citrus oil, bay oil, anise oil, eucalyptus oil, thyme oil, cedar leaf oil, nutmeg oil, sage oil, bitter almond oil, cassia oil), or combinations thereof, preferably strawberry flavor at about 0.1-2.0% w/v.

7. The composition of any one of claims 2 to 6, wherein the chelating agent is selected from disodium edetate (EDTA), tartaric acid, malic acid, citric acid, or combinations thereof.

8. The composition of any one of claims 2 to 7, wherein the antioxidant is selected from butylated hydroxyanisole (BHA), sodium metabisulfite, butylated hydroxytoluene (BHT), tocopherol, ascorbyl palmitate, ascorbic acid, sodium sulfite, sodium thiosulfate, propyl gallate, or combinations thereof, preferably BHA or sodium metabisulfite.

9. The composition of any one of claims 2 to 8, wherein the wetting agent is selected from sodium lauryl sulfate, sorbitan esters, polysorbates, lecithins, oleic acid and salts thereof, propylene glycol monostearate, glycerol monostearate, ethoxylated mono- and diglycerides, sucrose fatty acid esters, ethoxylated triglycerides, polyoxyethylated hydrogenated castor oil, sterols, or combinations thereof, preferably polysorbate 80 at a concentration of 0.01-0.5% w/v.

10. The composition of any one of claims 2 to 9, wherein the pH-adjusting agent is selected from hydrochloric acid, nitric acid, phosphoric acid, sulfuric acid, acetic acid, citric acid, malic acid, lactic acid, propionic acid, tartaric acid, ammonia solutions, monoethanolamine, diethanolamine, triethanolamine, meglumine, sodium acetate, sodium citrate, potassium bicarbonate, potassium citrate, potassium hydroxide, sodium bicarbonate, sodium borate, sodium hydroxide, or combinations thereof, preferably hydrochloric acid.

11. The composition of any one of claims 1 to 10, wherein the pH is maintained more preferably between 2.5 and 4.5 for optimal stability.

12. The composition of any one of claims 1 to 11, wherein the solution is stored in a light-protective container to minimize API degradation.

13. The composition of any one of claims 1 to 12, wherein the formulation remains free from visible precipitation or turbidity over the storage period.

14. A method for preparing a stable pharmaceutical oral solution composition comprising sitagliptin phosphate, the method comprising:
(a) dissolving the preservative, sweetener, and flavoring agent in purified water under continuous stirring;
(b) optionally adding the wetting agent;
(c) adding the pH-adjusting agent or a portion thereof;
(d) adding sitagliptin phosphate under controlled temperature conditions (20-35°C), preferably 15-25°C;
(e) optionally homogenizing the mixture to ensure uniform dissolution;
(f) adjusting the pH to the target value, if necessary;
(g) adjusting the final volume with purified water;
(h) optionally filtering the solution through a 1-10 µm pore filter; and
(i) filling the solution into appropriate containers.

15. The method of claim 14, wherein the solution is filled into light-protective containers under nitrogen flushing.
